# EUROPEAN PATENT SPECIFICATION

(11) **EP 0 873 732 B1**
(45) Date of publication and mention of the grant of the patent: **29.08.2001**
(21) Application number: 98303167.5
(22) Date of filing: 24.04.1998
(51) Int. Cl.: A61F 2/06, A61L 27/00

(54) **Coated endovascular stent**
Beschichteter endovaskulärer Stent
Extenseur endovasculaire muni d'un revêtement

(30) Priority: 24.04.1997 US 847763
(43) Date of publication of application: 28.10.1998
(73) Proprietor: Advanced Cardiovascular Systems, Inc., Santa Clara, CA 95054-8167 (US)
(72) Inventor: Yan, John J., Los Gatos, California 95032 (US); Chan, Randy, San Jose, California 95131 (US)
(74) Representative: McLeish, Nicholas Alistair Maxwell

(56) References cited:
- EP-A- 0 649 637
- EP-A- 0 701 802
- EP-A- 0 716 836
- EP-A- 0 756 853
- WO-A-97/10011
- US-A- 5 356 433

## Description

The present invention relates to endovascular stents and, more particularly pertains, to coatings that are applied to stents in order to reduce thrombogenicity post-implantation.

Stents are implanted within blood vessels in an effort to maintain the patency of the blood vessels by preventing collapse of the lumen and/or by impeding restenosis, a renewed blockage of the vessel such as might follow an angioplasty procedure performed to break up or dilate a previous blockage. Unfortunately, the presence of a foreign object in the path of the blood flow may have a thrombogenic effect. Therefore, it been found to be desirable to use various anti-coagulant drugs in an effort to provide an antithrombogenic effect and to reduce the likelihood of the development of restenosis.

A drug that has been found to be particularly effective for such purposes is heparin. By maintaining an effective concentration of the drug in and about the implantation site until the stent has been encapsulated by new body tissue, the risk of thrombogenesis can be mitigated substantially. To that end, various approaches have been used in the administration of heparin in connection with the deployment of stents.

While the systemic administration of heparin can cause the implantation site to be subjected to an effective level of heparin, such level of heparin also necessarily would be present in equal concentration throughout the rest of the patient's body, which can lead to undesirable side effects such as hard-to-control bleeding. Therefore, it has been recognized that a regimen in which the heparin is substantially constrained to the implantation site is far more desirable, An approach that has been devised to so constrain the drug involves coating or impregnating the stent with heparin. The heparin thereby is concentrated where it is needed most, but the concentration of the drug, and corresponding the impact of the drug on the patient's system, is localized.

For example, US-A-5,356,433 discloses a medical device or components of medical devices comprising metallic surfaces that have enhanced biocompatibility properties. The surfaces are prepared by a two-step procedure including covalently linking an organosilane having amine reactive sites with the surface of the metallic member, typically through a metal oxide thereof. Thereafter, a biologically active agent such as heparin is covalently linked to the organosilane coating. The two-step process is said to be particularly advantageous for preparing medical devices in the form of stents which need to be bent and flexed during implantation procedures.

Disadvantages associated with heretofore known heparinized stents include the limited shelf life of such devices, the fact the heparin is degraded when the stent is subjected to pre-deployment sterilization (either by heat or by a chemical solution, such as ethylene dioxide), the inability to alter the dosage to which the patient is subjected, and the inability to replenish any heparin that may be lost while the stent is in the process of being deployed. Additionally, the cost of bcrctofore known heparinized stent devices has been very high, as such cases necessarily include the expense incurred by reason of stringent regulatory requirements which often are associated with the provision of a drug-containing device.

The prior art has been unable to overcome these disadvantages and shortcomings and a new approach is needed to safely; effcctively, and economically deliver heparin to an implantation site.

The present invention provides for the coating of an implantable endovascular device to facilitate the subsequent loading of heparin onto its surface. Loading of the heparin is achieved in vitro just prior to implantation.

As a result of this timing of the coating process, the coated device has a shelf-life that is considerably longer than the shelf-life of pre-coated heparin-containing devices, the need for special handling and sterilization procedures associated with pre-coated heparin-containing devices is obviated, and the dosage of heparin readily can be tailored to requirements of an individual,

An additional advantage provided by a device according to the invention is that the device is not subject to the requirements often imposed by government agencies on drug-containing devices.

More particularly, the present invention provides a packaged, heparin-devoid stent, comprising:
a support structure that is implantable within the vasculature of a patient; and a coating deposited on said support structure; wherein such coating is formed of one or more materials that adhere to said stent structure and that provide functional groups that are capable of attracting heparin and with which heparin forms bonds when such material is exposed to a solution containing heparin.

Preferably, the coating materials provide ionic bonds with the stent surfaces. The functional groups incorporated in the coating may include primary,
secondary, and/or tertiary amines or other functionalities such as carboxyl groups.

The heparin-attracting coating may be applied so as to encapsulate the entire stent or, alternatively, to cover only selected surfaces thereof. By limiting coverage to only the inner surface of the stent, i.e, the surface that is directly exposed to the flow of blood following stent-deployment, a much higher level of heparin can be loaded onto the stent than would be considered safe if the same level were to be in direct contact with the vessel wall. Thus, a toxic effect on the vessel wall is avoided, but the blood in the vicinity of the device nonetheless is exposed to an effective concentration of heparin. Alternatively, it may be deemed sufficient to coat only the ends of the stent, i.e., at the post-deployment locations at which the disturbance of blood flow is greatest and where thromboses are most likely to occur.

The coating may be applied by different processes such as by dipping, spraying or molding. The preferred method is by plasma deposition wherein a base layer, the base layer selected for its ability to adhere to the stent surface, first is deposited on the stent followed by the deposition of a top layer thereon, which is selected for its ability to bond to the base layer and to contribute the appropriate functional groups for bonding to the heparin.

The present invention also provides an invitro method of preparing an endovascular stent suitable for delivering heparin to a specific site within the vasculature of a patient, comprising the steps of: providing an implantable support structure; depositing a coating thereon that adheres to said support structure and that includes functional groups that are capable of attracting heparin and with which heparin forms bonds when the coating is exposed to a solution containing heparin; sterilising and packaging said support structure having said coating deposited thereon;removing said coated support structure from its packaging; and exposing said coated support structure to a heparin-containing solution.

Other features and advantages of embodiments of the invention will become apparent from the following detailed description which, when taken in conjunction with the accompanying drawings, illustrates, by way of example, the principles of the invention.

### BRIEF DESCRIPTION OF THE DRAWING

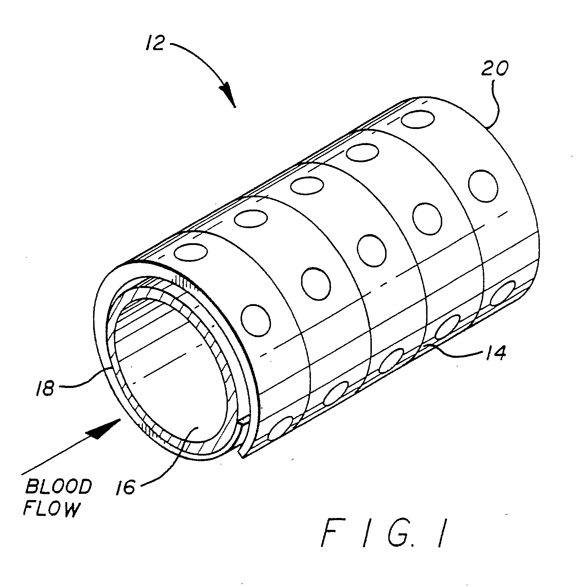

FIGURE 1 is a perspective view of an implantable stent.

### DETAILED DESCRIPTION OF THE PREFERRED EMBODIMENTS

A wide variety of differen: stent configurations have been devised to address various issues implicated by the intended use and desired function of the devices. Vanous materials have been used in constructing stents which include metals and polymers. Both the degree of turbulence caused by a particular stent configuration when subjected to blood flow and the material from which stent is constructed both affect the degree of thrombogenicity that can he experienced post-deployment. The present embodiment provides a coating for stents to which heparin becomes attached thus tending to reduce or eliminate the formation of thrombosis. Moreover, the nature of the coating is such that heparin can be loaded into it immediately before the implantation procedure.

A stent coating according to the present embodiment adheres to the stent surface and has functional groups that attract heparin and to which heparin bonds. Functional groups that are known to have the requisite affinity for heparin include primary, secondary, and tertiary amines. Primary amines are preferred, due to the exceptional affinity of the same for heparin. Alternatively, carboxyl groups may be used. The functional groups must include positively charged ions that serve to attract the negatively charged ions associated with the heparin. This attraction facilitates the formation of an ionic bond.

The coating can be applied by different processes such as by dipping, spraying, molding or by plasma deposition. Plasma deposition is preferred. In a first step of plasma deposition, a base layer or primer is laid down on the stent surface which prepares the surface to receive the substance containing the beparin-attracting-functional groups. In the preferred embodiment, a metallic stent is first plasma deposited with methane gas, which leaves a film on the surface of the stent. The methane molecules in the film are oriented with the carbon atoms against the stent and the hydrogen atoms are exposed. A top layer, that includes the desired functional groups then is deposited on the base layer. The second layer may be formed by the plasma deposition of ammonia gas, to leave the primary amine functional groups extending from the stent surface. Other chemicals such as alkylamine, nitrile compounds or amine-containing monomers also can be used to plasma deposit amine functional groups on the surface. When a mixture of primary, secondary, and tertiary amines is deposited by such methods, it is preferred that the primary amines constitute a greater percentage of the mixture than do the secondary and tertiary amines due to greater affinity of the primary amines for heparin. Alternatively, the deposition of carboxyl functional groups can be accomplished by the plasma deposition of monomers, such as methyl methacrylate or acrylic acid.

The thickness of the stent coating which results should be 0.0254 mm (0.001 inch) or less while a thickness of less than 1 micron is preferred. Although it may be desirable to have a uniform concentration of functional groups extending from the coated surface, it is not critical to the desired function of the coating. On the other hand, a concentration of at least 54 picamoles/stent must be achieved in order to ensure that heparin will attach to the coating at an effective level.

The coating may be applied to the entire stent or only to selected surfaces thereon. Figure 1 generally illustrates a stent 12 in the deployed state and serves to identify the surface 14, which faces the wall of the vessel into which the stent is implanted, the surface 16, which is exposed to the flow of blood through the stem, the "upstream" edge of the stent 18, and the "downstream" edge 20, which sequentially are exposed to the blood as it courses throughout the affected vessel. By coating only the surfaces facing the blood flow, a concentration of heparin can be loaded thereon that otherwise would be toxic to the vessel wall tissue if it were to be present on the surfaces that are in direct contact with the vessel wall post-deployment. Alternatively, it may be sufficient to exclusively coat the upstream and/or downstream edges of the stent for a particular stent configuration implanted in a particular patient, as thrombosis is most likely to occur at such interfaces due to the turbulence induced by the presence of the stent in the path of the flow of blood.

After the coating process is completed, the coated stent can be cleaned and sterilized and appropriately packaged for long-term storage. Due to the absence of any degradable drugs or substances on the stent, a fairly extended useful shelf-life can be expected.

In use, the physician pre-treats the stent prior to implantation by flushing it with, for example, a heparinized saline solution. In this way, the physician can easily and precisely adjust the heparin level by controlling the concentration of the heparin in the saline solution and/or controlling the time over which the stent and its coating are exposed to the heparin.

While a particular embodiment of the invention has been illustrated and described, it also will be apparent to those skilled in the art that various modifications can be made without departing from the scope of the invention as defined in the appended claims.

## Claims

1. A packaged, heparin-devoid stent, comprising:
a support structure that is implantable within the vasculature of a patient; and
a coating deposited on said support structure, wherein such coating is formed of one or more materials that adhere to said stent structure and that provide functional groups that are capable of attracting heparin and with which heparin forms bonds when such material is exposed to a solution containing heparin.

2. The endovascular stent of claim 1, wherein said heparin-containing solution comprises heparinized blood.

3. The endovascular stent of claim 1, wherein said heparin-containing solution comprises heparinized saline.

4. The endovascular stent of claim 1, wherein said coating is deposited on the entire support structure.

5. The endovascular stent of claim 1, wherein said support structure is configured such that, upon implantation in a blood vessel, such support structure has surfaces that face the vessel walls, surfaces that face the blood flow, and has an upstream edge and a downstream edge, said coating being exclusively deposited on said surface facing said blood flow.

6. The endovascular stent of claim 1, wherein said support structure is configured, such that upon implantation in a blood vessel, said support structure has surfaces that face the vessel walls, surfaces that face the blood flow, and has an upstream edge and a downstream edge, said coating being exclusively deposited on said upstream and downstream edges.

7. The endovascular stent of claim 1, wherein said bonds that the heparin attracting functional groups are capable of forming with heparin are ionic boards.

8. The endovascular stent of claim 7, wherein said functional groups comprise a primary amine.

9. The endovascular stent of claim 7, wherein said functional groups comprise a secondary amine.

10. The endovascular stent of claim 7, wherein the said functional groups comprise a tertiary amine.

11. The endovascular stent of claim 7, wherein said functional groups comprise carboxyl groups.

12. The endovascular stent of claim 1, wherein said coating comprises a base layer, wherein said base layer is selected for its ability to adhere to said support structure, and a top layer, said top layer being selected for its ability to bond to said base layer and including said functional groups that are capable of attracting heparin and with which heparin forms bonds when such material is exposed to a solution containing heparin.

13. The endovascular stent of claim 12, wherein said bonds that the heparin attracting functional groups are capable of forming with heparin are ionic bonds.

14. The endovascular stent of claim 12, wherein said base layer is hydrophobically attached to said support structure.

15. The endovascular stent of claim 12, wherein said top layer is covalently bonded to said base layer.

16. An invitro method of preparing an endovascular stent suitable for delivering heparin to a specific site within the vasculature of a patient, comprising the steps of:
providing an implantable support structure;
depositing a coating thereon that adheres to said support structure and that includes functional groups that are capable of attracting heparin and with which heparin forms bonds when the coating is exposed to a solution containing heparin;
sterilising and packaging said support structure having said coating deposited thereon;
removing said coated support structure from its packaging; and
exposing said coated support structure to a heparin-containing solution.

17. The method of claim 16, wherein said coating is deposited on the entire support structure.

18. The method of claim 16, wherein said support structure is configured such that upon implantation in a blood vessel, said support structure has surfaces that face the vessel walls, surfaces that face the blood flow and an upstream edge and a downsteam edge, said coating being deposited exclusively on said surfaces that face said blood flow.

19. The method of claim 16, wherein said support structure is configured such that upon implantation in a blood vessel, such support structure has surfaces that face the vessel walls, surfaces that face the blood flow and an upstream edge and a downstream edge, said coating being exclusively deposited on said upsteam and downsteam edges.

20. The method of claim 16, wherein said coating is deposited on said support structure by dipping.

21. The method of claim 16, wherein said coating is deposited on said structure by spraying.

22. The method of claim 16, wherein said coating is deposited on said support structure by molding.

23. The method of claim 16, wherein said coating is deposited on said support structure by plasma deposition.

24. The method of claim 16, wherein said coating is deposited by first depositing a base layer, selected for its ability to adhere to said support structure and then depositing thereon a top layer selected for its ability to bond to said base layer and which includes said functional groups that are capable of attracting heparin and with which heparin forms bonds when the coating is exposed to a solution containing heparin.

## Patentansprüche

1. Verpackter, heparinloser Stent, umfassend:
eine Trägerstruktur, die in dem Gefäßsystem eines Patienten implantierbar ist; und
eine Beschichtung, die auf die Trägerstruktur aufgetragen ist, wobei die Beschichtung aus einem oder mehreren Materialien gebildet ist, die an der Stentstruktur anhaften und die funktionelle Gruppen vorsehen, die in der Lage sind, Heparin anzuziehen, und mit denen Heparin Bindungen bildet, wenn dieses Material einer heparinhaltigen Lösung ausgesetzt wird.

2. Endovaskulärer Stent nach Anspruch 1, worin die heparinhaltige Lösung heparinisiertes Blut aufweist.

3. Endovaskulärer Stent nach Anspruch 1, worin die heparinhaltige Lösung heparinisierte Salzlösung aufweist.

4. Endovaskulärer Stent nach Anspruch 1, worin die Beschichtung auf die gesamte Trägerstruktur aufgetragen ist.

5. Endovaskulärer Stent nach Anspruch 1, wobei die Trägerstruktur derart konfiguriert ist, dass beim Implantieren in ein Blutgefäß diese Trägerstruktur Oberflächen aufweist, die zu den Gefäßwänden weisen, Oberflächen, die zu dem Blutfluss weisen, und einen stromaufwärtigen Rand und einen stromabwärtigen Rand aufweist, wobei die Beschichtung ausschließlich auf die zu dem Blutfluss weisende Oberfläche aufgetragen ist.

6. Endovaskulärer Stent nach Anspruch 1, wobei die Trägerstruktur derart konfiguriert ist, dass beim Implantieren in ein Blutgefäß diese Trägerstruktur Oberflächen aufweist, die zu den Gefäßwänden weisen, Oberflächen, die zu dem Blutfluss weisen, und einen stromaufwärtigen Rand und einen stromabwärtigen Rand aufweist, wobei die Beschichtung ausschließlich auf den stromaufwärtigen und den stromabwärtigen Rand aufgetragen ist.

7. Endovaskulärer Stent nach Anspruch 1, worin die Bindungen, die in der Lage sind, die heparinanziehenden funktionellen Gruppen mit Heparin zu bilden, lonenbindungen sind.

8. Endovaskulärer Stent nach Anspruch 7, worin die funktionellen Gruppen ein primäres Amin aufweisen.

9. Endovaskulärer Stent nach Anspruch 7, worin die funktionellen Gruppen ein sekundäres Amin aufweisen.

10. Endovaskulärer Stent nach Anspruch 7, worin die funktionellen Gruppen ein tertiäres Amin aufweisen.

11. Endovaskulärer Stent nach Anspruch 7, worin die funktionellen Gruppen Carboxylgruppen aufweisen.

12. Endovaskulärer Stent nach Anspruch 1, worin die Beschichtung eine Basisschicht aufweist, wobei die Basisschicht nach ihrer Fähigkeit zur Anhaftung an der Trägerstruktur ausgewählt ist, sowie eine Deckschicht, wobei die Deckschicht nach ihrer Fähigkeit zur Bindung an der Basisschicht ausgewählt ist und die funktionellen Gruppen enthält, die in der Lage sind, Heparin anzuziehen, und mit denen Heparin Bindungen bildet, wenn dieses Material einer heparinhaltigen Lösung ausgesetzt wird.

13. Endovaskulärer Stent nach Anspruch 12, worin die Bindungen, die in der Lage sind, die heparinanziehenden funktionellen Gruppen mit dem Heparin zu bilden, lonenbindungen sind.

14. Endovaskulärer Stent nach Anspruch 12, worin die Basisschicht an der Trägerstruktur hydrophob angebracht ist.

15. Endovaskulärer Stent nach Anspruch 12, worin die Deckschicht mit der Basisschicht kovalent gebunden ist.

16. In vitro-Verfahren zur Herstellung eines endovaskulären Stents, der zur Ausgabe von Heparin zu einer bestimmten Stelle innerhalb des Gefäßsystems eines Patienten geeignet ist, das die Schritte aufweist: Vorsehen einer implantierbaren Trägerstruktur;
Auftragen einer Beschichtung darauf, die an der Trägerstruktur anhaftet und die funktionelle Gruppen enthält, die in der Lage sind, Heparin anzuziehen, und mit denen Heparin Bindungen bildet, wenn die Beschichtung einer heparinhaltigen Lösung ausgesetzt wird;
Sterilisieren und Verpacken der Trägerstruktur mit der darauf aufgetragenen Beschichtung;
Entfernen der beschichteten Trägerstruktur aus ihrer Verpackung; und Aussetzen der beschichteten Trägerstruktur einer heparinhaltigen Lösung.

17. Verfahren nach Anspruch 16, worin die Beschichtung auf die gesamte Trägerstruktur aufgetragen ist.

18. Verfahren nach Anspruch 16, worin die Trägerstruktur derart konfiguriert ist, dass beim Implantieren in ein Blutgefäß die Trägerstruktur Oberflächen aufweist, die zu den Gefäßwänden weisen, Oberflächen, die zu dem Blutfluss weisen, sowie einen stromaufwärtigen Rand und einen stromabwärtigen Rand, wobei die Beschichtung ausschließlich auf die Oberflächen aufgetragen ist, die zu dem Blutfluss weisen.

19. Verfahren nach Anspruch 16, worin die Trägerstruktur derart konfiguriert ist, dass beim Implantieren in ein Blutgefäß diese Trägerstruktur Oberflächen aufweist, die zu den Gefäßwänden weisen, Oberflächen, die zu dem Blutfluss weisen, sowie einen stromaufwärtigen Rand und einen stromabwärtigen Rand, wobei die Beschichtung ausschließlich auf den stromaufwärtigen und den stromabwärtigen Rand aufgetragen ist.

20. Verfahren nach Anspruch 16, worin die Beschichtung durch Tauchen auf die Trägerstruktur aufgetragen ist.

21. Verfahren nach Anspruch 16, worin die Beschichtung durch Sprühen auf die Struktur aufgetragen ist.

22. Verfahren nach Anspruch 16, worin die Beschichtung durch Formgießen auf die Trägerstruktur aufgetragen ist.

23. Verfahren nach Anspruch 16, worin die Beschichtung durch Plasmaabscheidung auf die Trägerstruktur aufgetragen ist.

24. Verfahren nach Anspruch 16, worin die Beschichtung aufgetragen ist durch zuerst Auftragen einer Basisschicht, die nach ihrer Fähigkeit, auf der Trägerstruktur zu haften, gewählt ist, und dann Auftragen einer Deckschicht darauf, die nach ihrer Fähigkeit gewählt ist, an der Basisschicht zu binden, und die die funkionellen Gruppen enthält, die in der Lage sind, Heparin anzuziehen, und mit denen Heparin Bindungen bildet, wenn die Beschichtung einer heparinhaltigen Lösung ausgesetzt wird.

## Revendications

1. Endoprothèse dépourvue d'héparine, emballée, comprenant :
une structure de support que l'on peut implanter à l'intérieur du système vasculaire d'un patient ; et
un revêtement déposé sur ladite structure de support, dans laquelle ce revêtement est formé d'une ou de plusieurs matières qui adhèrent à ladite structure d'endoprothèse et qui fournissent des groupes fonctionnels qui peuvent attirer l'héparine et avec lesquels l'héparine forme des liaisons lorsque l'on expose cette matière à une solution contenant de l'héparine.

2. Endoprothèse vasculaire selon la revendication 1, dans laquelle ladite solution contenant de l'héparine comprend du sang héparinisé.

3. Endoprothèse vasculaire selon la revendication 1, dans laquelle ladite solution contenant de l'héparine comprend une solution saline héparinisée.

4. Endoprothèse vasculaire selon la revendication 1, dans laquelle ledit revêtement est déposé sur toute la structure de support.

5. Endoprothèse vasculaire selon la revendication 1, dans laquelle ladite structure de support est configurée de sorte que, lors de l'implantation dans un vaisseau sanguin, cette structure de support comporte des surfaces qui font face aux parois de vaisseau, des surfaces qui font face au flux sanguin, et un bord amont et un bord aval, ledit revêtement étant déposé exclusivement sur ladite surface faisant face au flux sanguin.

6. Endoprothèse vasculaire selon la revendication 1, dans laquelle ladite structure de support est configurée de sorte que, lors de l'implantation dans un vaisseau sanguin, ladite structure de support comporte des surfaces qui font face aux parois de vaisseau, des surfaces qui font face au flux sanguin, et comporte un bord amont et un bord aval, ledit revêtement étant déposé exclusivement sur lesdits bords amont et aval.

7. Endoprothèse vasculaire selon la revendication 1, dans laquelle lesdites liaisons que les groupes fonctionnels attirant l'héparine peuvent former avec l'héparine sont des liaisons ioniques.

8. Endoprothèse vasculaire selon la revendication 7, dans laquelle lesdits groupes fonctionnels comprennent une amine primaire.

9. Endoprothèse vasculaire selon la revendication 7, dans laquelle lesdits groupes fonctionnels comprennent une amine secondaire.

10. Endoprothèse vasculaire selon la revendication 7, dans laquelle lesdits groupes fonctionnels comprennent une amine tertiaire.

11. Endoprothèse vasculaire selon la revendication 7, dans laquelle lesdits groupes fonctionnels comprennent des groupes de carboxyles.

12. Endoprothèse vasculaire selon la revendication 1, dans laquelle ledit revêtement comprend une couche de base, dans laquelle ladite couche de base est choisie pour son aptitude à adhérer à ladite structure de support, et une couche superficielle, ladite couche superficielle étant choisie pour son aptitude à adhérer à ladite couche de base et comprenant lesdits groupes fonctionnels qui sont capables d'attirer l'héparine et avec lesquels l'héparine forme des liaisons lorsque l'on expose cette matière à une solution contenant de l'héparine.

13. Endoprothèse vasculaire selon la revendication 12, dans laquelle lesdites liaisons que les groupes fonctionnels attirant l'héparine peuvent former avec l'héparine sont des liaisons ioniques.

14. Endoprothèse vasculaire selon la revendication 12, dans laquelle ladite couche de base se fixe de manière hydrophobe à ladite structure de support.

15. Endoprothèse vasculaire selon la revendication 12, dans laquelle ladite couche superficielle se lie de manière covalente avec ladite couche de base.

16. Procédé in vitro de préparation d'une endoprothèse vasculaire approprié pour délivrer de l'héparine à un site spécifique à l'intérieur du système vasculaire d'un patient, comprenant les étapes dans lesquelles :
on utilise une structure de support pouvant être implantée ;
on dépose, sur celle-ci, un revêtement qui adhère à ladite structure de support et qui comprend des groupes fonctionnels qui peuvent attirer l'héparine et avec lesquels l'héparine forme des liaisons lorsque l'on expose le revêtement à une solution contenant de l'héparine ;
on stérilise et emballe ladite structure de support portant ledit revêtement déposé ;
on retire ladite structure de support revêtue de son emballage ; et
on expose ladite structure de support revêtue à une solution contenant de l'héparine.

17. Procédé selon la revendication 16, dans lequel on dépose ledit revêtement sur toute la structure de support.

18. Procédé selon la revendication 16, dans lequel ladite structure de support est configurée de sorte que, lors de l'implantation dans un vaisseau sanguin, ladite structure de support comporte des surfaces qui font face aux parois de vaisseau, des surfaces qui font face au flux sanguin et un bord amont et un bord aval, ledit revêtement étant déposé exclusivement sur lesdites surfaces qui font face audit flux sanguin.

19. Procédé selon la revendication 16, dans lequel ladite structure de support est configurée de sorte que, lors de l'implantation dans un vaisseau sanguin, cette structure de support comporte des surfaces qui font face aux parois de vaisseau, des surfaces qui font face au flux sanguin et un bord amont et un bord aval, ledit revêtement étant déposé exclusivement sur lesdits bords amont et aval.

20. Procédé selon la revendication 16, dans lequel on dépose, par immersion, ledit revêtement sur ladite structure de support.

21. Procédé selon la revendication 16, dans lequel on dépose, par pulvérisation, ledit revêtement sur ladite structure.

22. Procédé selon la revendication 16, dans lequel on dépose, par moulage, ledit revêtement sur ladite structure de support.

23. Procédé selon la revendication 16, dans lequel on dépose, par dépôt au plasma, ledit revêtement sur ladite structure de support.

24. Procédé selon la revendication 16, dans lequel on dépose ledit revêtement en déposant d'abord une couche de base, choisie pour son aptitude à adhérer à ladite structure de support, et l'on dépose ensuite, sur celle-ci, une couche superficielle choisie pour son aptitude à adhérer à ladite couche de base et qui inclut des groupes fonctionnels qui peuvent attirer l'héparine et avec lesquels l'héparine forme des liaisons lorsque l'on expose le revêtement à une solution contenant de l'héparine.
